# FASCICULE DE BREVET EUROPEEN

(11) **EP 2 536 370 B1**
(45) Date de publication et mention de la délivrance du brevet: **07.01.2015**
(21) Numéro de dépôt: 11708543.1
(22) Date de dépôt: 09.02.2011
(51) Int. Cl.: A61F 13/06, A61F 13/08, A47G 25/90

(54) **ORTHÈSE COMPRESSIVE TUBULAIRE**
RÖHRENFÖRMIGEN DRUCK-ORTHESE
COMPRESSIVE TUBULAR,ORTHOSIS

(30) Priorité: 17.02.2010 FR 1051119
(43) Date de publication de la demande: 26.12.2012
(73) Titulaire: Thuasne, 92300 Levallois Perret (FR)
(72) Inventeur: CONVERT, Reynald, F-42800 Saint Martin La Plaine (FR); GERARD, Marie, F-92150 Suresnes (FR); COTTE, Alain, F-42100 Saint Etienne (FR)
(74) Mandataire: Balesta, Pierre
(86) Numéro de dépôt international: PCT/FR2011/050271
(87) Numéro de publication internationale: WO 2011/101578

(56) Documents cités:
- WO-A1-91/05498
- WO-A1-99/44548
- WO-A1-03/096851
- WO-A1-2008/006142
- FR-A1- 2 775 431

## Description

La présente invention concerne une orthèse compressive tubulaire destinée à améliorer le soutien mécanique de la région du corps qu'elle recouvre ainsi que la proprioception.

La proprioception est l'aptitude du porteur à connaître les positions et les mouvements des parties de son corps dans l'espace, sans qu'il ait besoin de vérifier visuellement. Ceci permet notamment d'éviter les chutes et de blesser à nouveau l'articulation soutenue.

Les orthèses compressives sont difficiles à placer correctement sur l'articulation à renforcer. Bien souvent la préconisation d'une orthèse, par exemple une genouillère, est consécutive à une blessure, de sorte que l'usager ne peut mettre en flexion ou en extension complètement l'articulation en question, et est ainsi gêné dans la pose de son orthèse.

On connaît des articles chaussants classiques, du type chaussette, sans effet de compression, comportant des boucles se projetant des côtés ou de la bordure supérieure desdits articles chaussants et destinées à faciliter l'enfilage. Ces boucles sont généralement tricotées étant intégrées lors du tricotage de l'article chaussant. Ces boucles se projetant des articles chaussants présentent l'inconvénient qu'elles risquent de s'accrocher sur des objets environnants, en particulier lors de la pratique d'un sport. De plus, ces boucles sont inesthétiques, créant par exemple des protubérances sous les vêtements. Enfin, ces boucles sont peu résistantes et la traction exercée sur ces dernières pour l'enfilage de l'article chaussant risque de les détériorer ainsi que les zones de l'article chaussant sur lesquelles elles sont rapportées.

On connaît également des dispositifs tubulaires de compression, du type bas de contention, comportant un premier bas ayant un très faible coefficient de friction et un second bas à effet de compression. Le premier bas est tout d'abord disposé sur la jambe, puis le second bas est disposé sur le premier bas, l'enfilage du second bas étant facilité par la glissance du second bas sur le premier bas. Puis, le premier bas est tiré, par l'usager, sous le second bas en sorte que seul le second bas reste disposé sur la jambe. Ces dispositifs sont complexes et onéreux à fabriquer, et ne peuvent généralement être mis en place par des personnes ayant une articulation de la jambe dont la mobilité est réduite.

La présente invention a pour but de proposer une orthèse compressive tubulaire, en particulier une genouillère, comportant au moins un organe de préhension facilitant son enfilage, esthétique, solide, peu onéreuse à fabriquer, facile à mettre en oeuvre, confortable et ne risquant pas de s'accrocher à des objets environnants.

La présente invention a ainsi pour objet une orthèse compressive tubulaire, qui présente un bord supérieur délimitant une première zone tricotée de compression et qui comprend, à proximité dudit bord supérieur, au moins un organe de préhension facilitant l'enfilage de ladite orthèse. De manière caractéristique, l'organe de préhension est une seconde zone tricotée de préhension, disposée sous le bord supérieur et présentant une densité de mailles supérieure à la densité de mailles dans la première zone de compression.

On comprend par densité de mailles, le nombre de colonnes et/ou de rangées de mailles par centimètre ou par pouce.

On entend par compression, toute partie ou zone de l'orthèse apte à exercer une force de compression [mmHg], de préférence supérieure à 7 mmHg.

La force de compression locale exercée sur un membre par un article de compression est fonction notamment des caractéristiques de force-allongement dudit article.

La pression exercée sur un membre se calcule par la loi de Laplace suivante : P [Pa ou mmHg] = (T [N] X n / (L [m] X R [m]).

P représente la pression exercée en un point donné du membre considéré.

T est la tension, exprimée en Newtons, exercée par ledit article ou une zone de celui-ci, lorsqu'il est enfilé sur le membre inférieur ou supérieur ou encore le tronc.

R est le rayon de courbure au point considéré du membre inférieur ou supérieur ou encore du tronc.

L est la longueur ou hauteur dudit article ou d'une zone de celui-ci.

n représente le nombre d'épaisseurs de matières textiles dudit article ou dans une zone de celui-ci.

L'orthèse compressive peut être formée d'une seule ou de plusieurs premières zones de compression tricotées simultanément.

De préférence, l'orthèse est formée par tricotage sur un métier rectiligne en sorte de former un panneau dont les bords opposés sont assemblés pour former un tube.

Avantageusement, l'organe de préhension est formé par la seconde zone tricotée, disposée entre la première zone tricotée de compression et le bord supérieur, de sorte qu'il est parfaitement intégré dans l'orthèse et vient en contact avec le corps de l'usager et contribue ainsi au soutien mécanique et à l'effet de proprioception apportés par l'orthèse. L'organe de préhension présente ainsi également l'avantage d'être résistant aux tractions exercées par l'usager.

De plus, la densité de mailles étant plus importante dans la seconde zone que la densité de mailles dans la première zone, cette disposition entraîne dans la seconde zone une légère surépaisseur ainsi qu'un toucher rigide facilitant l'identification et la préhension de celle-ci.

De préférence, le bord supérieur comporte une ondulation latérale en saillie localisée au-dessus de la dite seconde zone. Cette disposition améliore l'identification et facilite la préhension par l'usager de l'organe de préhension.

Ladite ondulation latérale est agencée sur l'orthèse en sorte de se retrouver sur le côté interne ou externe de l'articulation à soutenir lorsque l'orthèse est portée.

Ce type de dispositif facilitant la préhension peut également s'appliquer à d'autres types d'orthèses tel que des chevillières, des orthèses de poignet ou des orthèses de coude par exemple. Il peut être également utilisé pour des bas, des chaussettes ou des manchons de compression.

L'orthèse tubulaire selon l'invention peut être utilisée telle quelle ou être intégrée par tricotage ou par couture ou n'importe quels moyens équivalents dans un autre article tel qu'une chaussette.

Dans une variante, le bord supérieur présente deux ondulations latérales en saillie et l'orthèse comporte deux secondes zones de préhension localisées sous les deux dites ondulations et faisant office d'organes de préhension.

Les secondes zones de préhension sont de préférence disposées sur l'orthèse à l'opposé l'une de l'autre en sorte de se retrouver en fonctionnement sur les côtés interne et externe de l'articulation à protéger.

Dans une variante, la ou les secondes zones comprennent un fil de maille élastique et un fil de maille non élastique.

Les secondes zones ne comprenant pas de fil de trame élastique n'exercent pas de force de compression sur l'articulation de l'usager avec laquelle elles sont en contact en fonctionnement. Par comparaison, la première zone de compression comprend nécessairement un fil de trame élastique afin d'assurer son effet compressif. Ainsi, la première zone comporte des fils tricotés dont la somme des titrages (dtex), de chacun desdits fils, est supérieure à la somme des titrages des fils tricotés dans les secondes zones de sorte que les secondes zones sont moins rigides au toucher que la première zone de compression. Il est possible d'augmenter le titrage du fil de maille non élastique dans la ou les secondes zones en sorte d'augmenter leur rigidité.

Dans une variante, la ou les secondes zones comprennent un fil de trame élastique en sorte d'exercer un effet de compression.

Avantageusement, les secondes zones assurent non seulement la fonction d'organes de préhension mais également les fonctions assurées par la première zone de compression, notamment un soutien mécanique.

Dans une variante, la densité de mailles dans la ou les secondes zone(s) est supérieure ou égale à deux fois la densité de mailles dans la première zone de compression à laquelle elle reliée par tricotage.

Le demandeur a établi que pour que les effets décrits ci-dessus soient optimisés, en terme de rigidité notamment, le ratio ci-dessus devait être respecté.

Dans une variante, le bord supérieur est tricoté avec un seul type de fil de maille, de préférence non élastique, par tricotage sur un selon le schéma de mailles suivant :
- une première rangée de mailles tricotées sur toutes les aiguilles de la fonture arrière ;
- une deuxième rangée de mailles tricotées sur toutes les aiguilles de la fonture avant.

Le bord supérieur est tricoté de préférence à partir d'un seul type de fil de maille, de préférence non élastique.

Dans une variante, la première zone de compression et la ou les seconde(s) zone(s) sont tricotées selon un même schéma de mailles.

Cette disposition simplifie la fabrication de l'orthèse et diminue ainsi son coût.

Dans une variante, la première zone de compression, et éventuellement la ou les seconde(s) zone(s), sont tricotées selon le schéma de mailles suivant :
- une première rangée sans mailles selon laquelle est inséré un fil de trame élastique ;
- une seconde rangée de mailles tricotées en côtes 1 et 1, de préférence avec un fil de maille non élastique ;
- une troisième rangée de mailles tricotées sur la fonture arrière, de préférence avec un fil de maille élastique.

Dans ce cas, la différenciation de densité de mailles entre la première zone et la ou les secondes zones est obtenue en multipliant la répétition des schémas de mailles dans chaque zone. Ainsi, ce schéma de mailles est répété 16 fois dans la ou les secondes zones alors qu'il est répété 8 fois dans la première zone.

Dans une variante, le fil de maille élastique a un titrage supérieur ou égal à deux fois le titrage du fil de maille non élastique, de préférence le fil de trame a un titrage supérieur ou égal à deux fois le fil de maille élastique.

Cette disposition permet d'obtenir une orthèse compressive élastique. Dans une variante, ladite orthèse comprend une troisième zone tricotée agencée en sorte de recouvrir, lorsque ladite orthèse est portée, au moins le creux poplité, et ladite troisième zone ne comprend pas de fil de trame élastique.

De préférence, la troisième zone est tricotée avec un fil de maille élastique et un fil de maille non élastique selon l'invention.

La troisième zone n'ayant pas d'effet de compression, elle ne risque pas de blesser par pincement ou de créer des phénomènes de striction ou d'étranglement dans cette zone du corps riche en vaisseaux sanguins et en nerfs et selon laquelle la peau est fine.

Dans une sous-variante, la troisième zone comprend une densité de mailles supérieure, de préférence supérieure ou égale à deux fois, la densité de mailles de la première zone de compression avec laquelle elle est reliée par tricotage.

Avantageusement, la troisième zone est formée intégralement lors du tricotage de l'orthèse compressive tubulaire de sorte qu'elle n'est pas rapportée par confection sur ladite orthèse. Cette disposition améliore le confort de l'usager en évitant le contact avec la peau de coutures rigides et abrasives.

De préférence, la troisième zone s'étend sur moins d'un quart de la circonférence de l'orthèse compressive tubulaire selon l'invention. Cette disposition assure le confort de l'usager tout en augmentant la surface de l'orthèse disponible pour une première zone de compression, améliorant ainsi le soutien mécanique et la proprioception.

Le ou les fils de maille, élastique(s) ou non, ainsi que le ou les fils de trame élastique(s) peuvent être des fils multifilamentaires ou des monofilaments simples ou guipés à l'aide d'un ou plusieurs fil(s) multifilamentaire(s), retordus ou non, ou d'un ou plusieurs monofilament(s).

La présente invention sera mieux comprise à la lecture d'un exemple de réalisation, cité à titre non limitatif, et illustré par les figures ci-après, annexées à la présente, et dans lesquelles :
- la figure 1 est une vue de côté d'un exemple d'orthèse compressive selon l'invention ;
- la figure 2 est une représentation d'un premier schéma de mailles ;
- la figure 3 est une représentation d'un second schéma de mailles ;
- la figure 4 est une vue de la face arrière de l'orthèse compressive représentée à la figure 1.

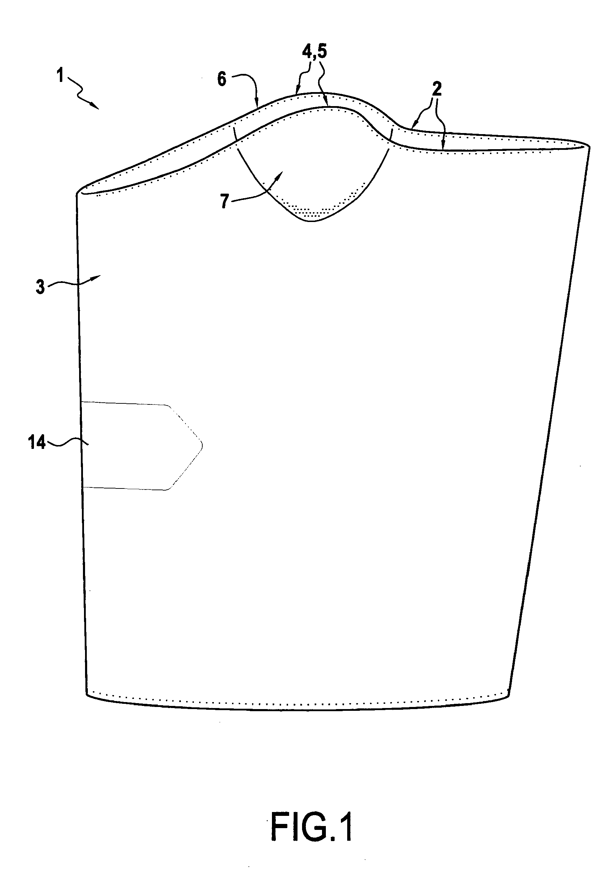

L'orthèse compressive tubulaire 1 représentée à la figure 1 est une genouillère ayant un bord supérieur 2 délimitant une première zone 3 tricotée de compression. Ledit bord supérieur 2 présente deux ondulations latérales en saillie 4,5 disposées à l'opposé l'une de l'autre, et en sorte de se retrouver sur les côtés interne et externe du genou de l'usager lorsque la genouillère est portée. L'orthèse 1 comprend à proximité dudit bord supérieur 2, deux secondes zones de préhension 6,7 localisées sous lesdites ondulations 4,5 et donc sous ledit bord supérieur 2. Ces secondes zones de préhension 6,7 font office d'organes de préhension.

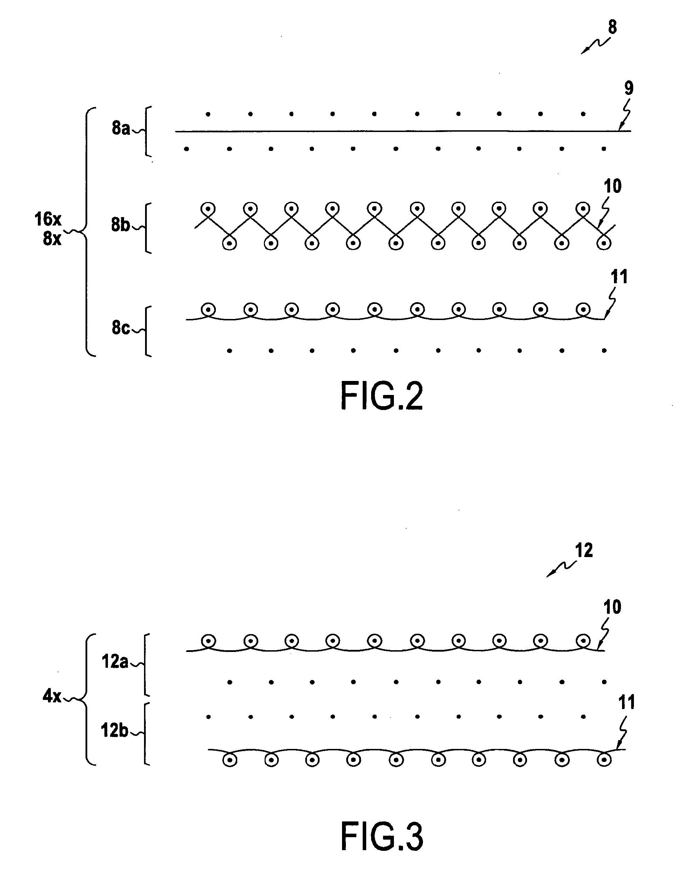

De préférence, les secondes zones de préhension 6,7 sont tricotées selon le même schéma de mailles que la première zone de compression 3. Le schéma de mailles 8, représentée à la figure 2, comprend :
- une première rangée 8a sans mailles selon laquelle est inséré un fil de trame élastique 9 ;
- une seconde rangée 8b de mailles tricotées en côtes 1 et 1, de préférence avec un fil de maille non élastique 10 ;
- une troisième rangée 8c de mailles tricotées sur la fonture arrière, de préférence avec un fil de maille élastique 11.

Les seconde 8b et troisième 8c rangées de mailles peuvent être inversées localement sur l'orthèse 1.

La première zone de compression 3 et les secondes zones de préhension 6,7 sont ainsi avantageusement tricotées lors d'une même étape de tricotage à partir d'un fil de maille élastique 11, d'un fil de maille non élastique 10 et d'un fil de trame élastique 9. Le schéma de mailles 8 est répété huit fois dans la première zone de compression 3 contre seize fois dans les secondes zones de préhension 6,7, ce qui contribue à obtenir une densité de mailles dans les secondes zones 6,7 supérieure ou égale à deux fois la densité de mailles dans la première zone de compression 3. Par ailleurs, la caractéristique selon laquelle la première zone de compression 3 comprend un fil de trame élastique 9 permet également de compresser et donc resserrer les colonnes et/ou rangées de mailles dans les secondes zones de compression 6,7 contribuant ainsi à augmenter la densité de mailles dans lesdites secondes zones.

Cette différenciation de la densité de mailles ainsi que la forme en saillie des secondes zones de préhension 6,7 facilitent le repérage et la préhension desdites secondes zones 6,7 par l'utilisateur.

De plus, les secondes zones 6,7 font parties intégrantes de l'orthèse 1 et ne se projettent pas de celle-ci évitant ainsi de s'accrocher, voire de se déchirer et contribue à l'effet de soutien mécanique et à l'effet de proprioception apportée par l'orthèse 1. Les secondes zones 6,7 comportant des fils de trame élastiques 9 contribuent également à l'effet compressif apporté par l'orthèse 1.

Il est possible également de supprimer le fil de trame élastique 9 dans les secondes zones 6,7 afin d'assouplir ces dernières.

Le bord supérieur 2 est tricoté en même temps que la première zone de compression 3 et les secondes zones de préhension 6,7, et selon le schéma de mailles 12 suivant, représenté à la figure 3 :
- une première rangée 12a de mailles tricotées sur toutes les aiguilles de la fonture arrière à partir d'un fil de maille non élastique 10 ;
- une deuxième rangée 12b de mailles tricotées sur toutes les aiguilles de la fonture avant à partir d'un fil de maille élastique 11.

Le schéma de mailles 12 est de préférence répété quatre fois au niveau dudit bord supérieur 2.

De préférence, l'orthèse 1 compressive est fabriquée intégralement par tricotage rectiligne d'un panneau, puis les bords latéraux opposés sont assemblés selon une couture 13 en sorte de former un tube.

L'orthèse 1 comprend également sur sa face postérieure 1b une troisième zone 14 tricotée agencée en sorte de recouvrir en fonctionnement le creux poplité. Cette troisième zone 14 comprend également un fil de maille élastique 11 et un fil de maille non élastique 10. La troisième zone 14 ne comprend pas de fil de trame élastique ce qui évite de blesser la zone du creux poplité fragile. La densité de mailles dans la troisième zone 14 est supérieure à celle de la première zone de compression 3, de préférence supérieure ou égale à deux fois celle de la première zone de compression 3. La troisième zone 14 est ainsi tricotée selon le schéma de mailles 8 représenté à la figure 2, excepté qu'elle ne comprend pas de fil de trame élastique 9 et que la seconde rangée 8b de mailles est inversée avec la troisième rangée 8c de mailles, tout comme dans la première zone de compression 3 entourant ladite troisième zone 14 à laquelle elle est liée par tricotage.

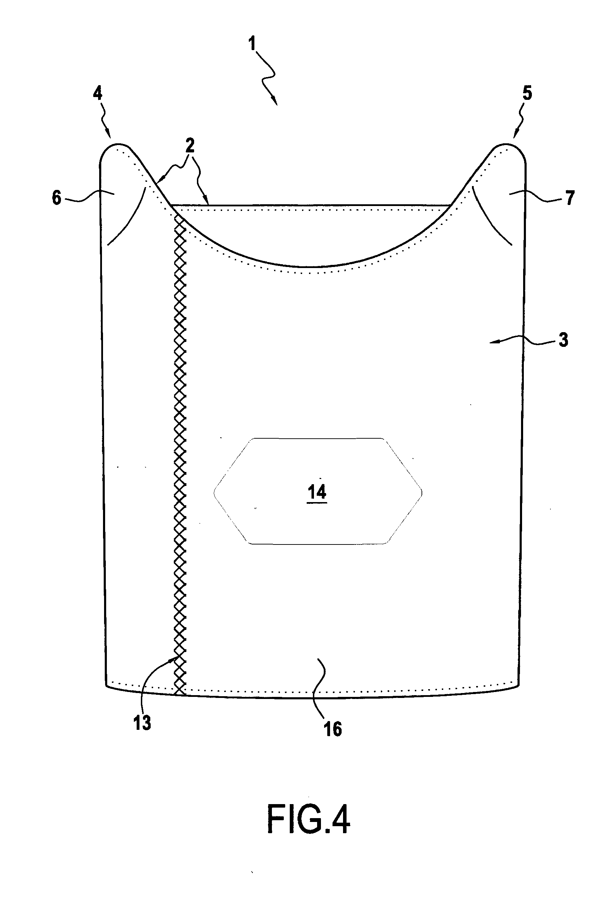

Dans cet exemple particulier, et telle que représentée sur les figures 1 et 4, la troisième zone 14 s'étend sur moins d'un quart de la circonférence de l'orthèse 1.

Dans la première zone de compression 3, les secondes zones de préhension 6,7 et la troisième zone 14 recouvrant le creux poplité, le fil de maille élastique 11 a un titrage supérieur au moins à deux fois celui du fil de maille non élastique 10, et le fil de trame 9 a un titrage supérieur ou égale à deux fois le titrage du fil de maille élastique 11. A titre d'exemple précis, le fil de maille non élastique 10 a un titrage de l'ordre de 150 dtex, le fil de maille élastique 11 a un titrage de l'ordre de 400 dtex et comporte un fil d'âme élastique double guipé par des fils non élastiques, et le fil de trame 9 a un titrage de l'ordre de 900 dtex et comporte un monofilament en élastique double guipé par des fils multifilamentaires non élastiques.

Il est possible d'augmenter la rigidité des secondes zones en augmentant le titrage du fil de maille élastique 11, en particulier du monofilament en âme élastique.

## Revendications

1. Orthèse (1) compressive tubulaire qui présente un bord supérieur (2) délimitant une première zone (3) tricotée de compression et qui comprend, à proximité dudit bord supérieur (2), au moins un organe de préhension facilitant l'enfilage de ladite orthèse **caractérisée en ce que** l'organe de préhension est une seconde zone tricotée de préhension (6,7), disposée sous le bord supérieur (2) et présentant une densité de mailles supérieure à la densité de mailles de la première zone de compression (3).

2. Orthèse (1) selon la revendication 1 **caractérisée en ce que** le bord supérieur (2) présente deux ondulations latérales en saillie (4,5) et **en ce que** l'orthèse comporte deux secondes zones de préhension (6,7) localisées sous les deux dites ondulations et faisant office d'organes de préhension.

3. Orthèse (1) selon l'une ou l'autre des revendications 1 et 2, **caractérisée en ce que** la ou les secondes zones (6,7) comprennent un fil de maille élastique (11) et un fil de maille non élastique (10).

4. Orthèse (1) selon la revendication 3, **caractérisée en ce que** la ou les secondes zones (6,7) comprennent un fil de trame élastique (9) en sorte d'exercer un effet de compression.

5. Orthèse (1) selon l'une quelconque des revendications 1 à 4, **caractérisée en ce que** la densité de mailles dans la ou les secondes zones (6,7) est supérieure ou égale à deux fois la densité de mailles dans la première zone (3) à laquelle elle reliée par tricotage.

6. Orthèse (1) selon l'une des revendications 1 à 5, caractérisée en en ce que le bord supérieur (2) est tricoté avec un seul type de fil de maille, de préférence non élastique (10), par tricotage selon le schéma de mailles (12) suivant :
- une première rangée (12a) de mailles tricotées sur toutes les aiguilles de la fonture arrière ;
- une deuxième rangée (12b) de mailles tricotées sur toutes les aiguilles de la fonture avant.

7. Orthèse (1) selon l'une des revendications 1 à 6, **caractérisée en ce que** la première zone de compression (3) et la ou les seconde zone(s) de préhension (6,7) sont tricotées selon un même schéma de mailles.

8. Orthèse selon l'une des revendications 1 à 7, **caractérisée en ce que** la première zone de compression (3), et éventuellement la ou les seconde(s) zone(s) de préhension (6,7), sont tricotées selon le schéma de mailles (8) suivant :
- une première rangée (8a) sans mailles selon laquelle est inséré un fil de trame élastique (9) ;
- une seconde rangée (8b) de mailles tricotées en côtes 1 et 1, de préférence avec un fil de maille non élastique (10) ;
- une troisième rangée (8c) de mailles tricotées sur la fonture arrière, de préférence avec un fil de maille élastique (11) ;
et **en ce que** les seconde (8b) et troisième (8c) rangées peuvent être inversées.

9. Orthèse (1) selon l'une des revendications 3 à 8, **caractérisée en ce que** le fil de maille élastique (11) a un titrage supérieur ou égal à deux fois le titrage du fil de maille non élastique (10), et **en ce que** le fil de trame (9) a un titrage supérieur ou égal à deux fois le titrage du fil de maille élastique (11).

10. Orthèse (1) selon l'une des revendications 1 à 9, **caractérisée en ce qu'**elle comprend une troisième zone (14) tricotée agencée en sorte de recouvrir lorsque ladite orthèse est portée au moins le creux poplité, et **en ce que** ladite troisième zone (14) ne comprend pas de fil de trame élastique (9).

11. Orthèse (1) selon la revendication 10, **caractérisée en ce que** la troisième zone (14) comprend une densité de mailles supérieure, de préférence supérieure ou égale à deux fois, la densité de mailles de la première zone (3) de compression avec laquelle elle est reliée par tricotage.

## Patentansprüche

1. Röhrenförmige Kompressionsorthese (1), die einen oberen Rand (2) aufweist, welcher einen ersten gestrickten Kompressionsbereich (3) begrenzt, und die in der Nähe des oberen Randes (2) wenigstens ein Greiforgan, welches das Anziehen der Orthese erleichtert, umfasst, **dadurch gekennzeichnet, dass** das Greiforgan ein zweiter gestrickter Greifbereich (6, 7) ist, der unter dem oberen Rand (2) angeordnet ist und eine Maschendichte aufweist, die größer als die Maschendichte des ersten Kompressionsbereichs (3) ist.

2. Orthese (1) nach Anspruch 1, **dadurch gekennzeichnet, dass** der obere Rand (2) zwei vorspringende seitliche Wellen (4, 5) aufweist und dass die Orthese zwei zweite Greifbereiche (6, 7) umfasst, die unter den beiden Wellen gelegen sind und als Greiforgane dienen.

3. Orthese (1) nach dem einen oder anderen der Ansprüche 1 und 2, **dadurch gekennzeichnet, dass** der oder die zweiten Bereiche (6, 7) einen elastischen Musterfaden (11) und einen nicht elastischen Musterfaden (10) umfassen.

4. Orthese (1) nach Anspruch 3, **dadurch gekennzeichnet, dass** der oder die zweiten Bereiche (6, 7) einen elastischen Schussfaden (9) umfassen, so dass eine Kompressionswirkung ausgeübt wird.

5. Orthese (1) nach einem der Ansprüche 1 bis 4, **dadurch gekennzeichnet, dass** die Maschendichte in dem oder den zweiten Bereichen (6, 7) größer als die oder gleich der zweifache(n) Maschendichte in dem ersten Bereich (3) ist, mit dem er durch Stricken verbunden ist.

6. Orthese (1) nach einem der Ansprüche 1 bis 5, **dadurch gekennzeichnet, dass** der obere Rand (2) mit einem einzigen Typ von Musterfaden, vorzugsweise nicht elastischen (10), durch Stricken nach dem folgenden Maschenschema (12) gestrickt ist:
- eine erste Reihe (12a) von auf allen Nadeln der hinteren Fontur gestrickten Maschen,
- eine zweite Reihe (12b) von auf allen Nadeln der vorderen Fontur gestrickten Maschen.

7. Orthese (1) nach einem der Ansprüche 1 bis 6, **dadurch gekennzeichnet, dass** der erste Kompressionsbereich (3) und der oder die zweite(n) Greifbereich(e) (6, 7) nach einem gleichen Maschenschema gestrickt sind.

8. Orthese nach einem der Ansprüche 1 bis 7, **dadurch gekennzeichnet, dass** der erste Kompressionsbereich (3) und eventuell der oder die zweite(n) Greifbereich(e) (6, 7) nach dem folgenden Maschenschema gestrickt sind:
- eine erste Reihe (8a) ohne Maschen, entlang der ein elastischer Schussfaden (9) eingefügt wird,
- eine zweite Reihe (8b) von Maschen, die in Rechts-Rechts-Bindungen, vorzugsweise mit einem nicht elastischen Musterfaden (10) gestrickt sind,
- eine dritte Reihe (8c) von Maschen, die auf der hinteren Fontur, vorzugsweise mit einem elastischen Musterfaden (11) gestrickt sind,
und dass die zweite (8b) und die dritte (8c) Reihe umgekehrt werden können.

9. Orthese (1) nach einem der Ansprüche 3 bis 8, **dadurch gekennzeichnet, dass** der elastische Musterfaden (11) eine Nummer größer als die oder gleich der zweifache(n) Nummer des nicht elastischen Musterfadens (10) aufweist und dass der Schussfaden (9) eine Nummer größer als die oder gleich der zweifache(n) Nummer des elastischen Musterfadens (11) aufweist.

10. Orthese (1) nach einem der Ansprüche 1 bis 9, **dadurch gekennzeichnet, dass** sie einen dritten gestrickten Bereich (14) umfasst, der so angeordnet ist, dass er, wenn die Orthese getragen wird, wenigstens die Kniekehle bedeckt, und dass der dritte Bereich (14) keinen elastischen Schussfaden (9) umfasst.

11. Orthese (1) nach Anspruch 10, **dadurch gekennzeichnet, dass** der dritte Bereich (14) eine Maschendichte aufweist, die größer, vorzugsweise größer als die oder gleich der zweifachen Maschendichte des ersten Kompressionsbereichs (3) ist, mit dem er durch Stricken verbunden ist.

## Claims

1. A tubular compressive orthosis (1) having an upper edge (2) delimiting a first knitted compression zone (3) and which, in the vicinity of the said upper edge (2), comprises at least one grip member facilitating the process of putting on the said orthosis, **characterized in that** the grip member is a second knitted grip zone (6,7) arranged underneath the upper edge (2) and having a stitch density greater than the stitch density of the first compression zone (3).

2. The orthosis (1) according to claim 1 **characterized in that** the upper edge (2) has two side protruding ripples (4,5) and **in that** the orthosis comprises two second grip zones (6,7) located underneath the two said ripples and acting as gripping members.

3. The orthosis (1) according to either of claims 1 and 2 **characterized in that** the second zone(s) (6, 7) comprise an elastic knit yarn (11) and a non-elastic knit yarn (10).

4. The orthosis (1) according to claim 3 **characterized in that** the second zone(s) (6, 7) comprise an elastic weft yarn (9) so as to apply a compressive effect.

5. The orthosis (1) according to any of claims 1 to 4 **characterized in that** the stitch density in the second zone(s) (6,7) is equal to or higher than twice the stitch density in the first zone (3) with which it is connected via the knit.

6. The orthosis (1) according to one of claims 1 to 5 **characterized in that** the upper edge (2) is knitted with a single type of knit yarn, preferably non-elastic (10), by knitting as per the following knit structure (12):
- a first row (12a) of stitches knitted on all the needles of the rear bed;
- a second row (12b) of stitches knitted on all the needles of the front bed.

7. The orthosis (1) according to one of claims 1 to 6 **characterized in that** the first compression zone (3) and the second grip zone(s) (6,7) are knitted following one same knit structure.

8. The orthosis according to one of claims 1 to 7 **characterized in that** the first compression zone (3) and optionally the second grip zone(s) (6, 7) are knitted as per the following knit structure (8):
- a first stitch-free row (8a) along which an elastic weft yarn is inserted (9);
- a second row (8b) of stitches knitted in 1/1 rib stitch preferably with a non-elastic knit yarn (10);
- a third row (8c) of stitches knitted on the rear bed, preferably with an elastic knit yarn (11);
and **in that** the second (8b) and third (8c) rows can be reversed.

9. The orthosis (1) according to one of claims 3 to 8 **characterized in that** the elastic knit yarn (11) has a yarn count equal to or higher than twice the yarn count of the non-elastic knit yarn (10) and **in that** the weft yarn (9) has a yarn count equal to or higher than twice the yarn count of the elastic knit yarn (11).

10. The orthosis (1) according to one of claims 1 to 9 **characterized in that** it comprises a third knitted zone (14) arranged so that, when the said orthosis is being worn, it covers at least the popliteal fossa and **in that** the said third zone (14) does not comprise an elastic weft yarn (9).

11. The orthosis (1) according to claim 10, **characterized in that** the third zone (14) has a greater stitch density, preferably equal to or more than twice the stitch density of the first compression zone (3) with which it is connected via the knit.
